(19) Europäisches Patentamt
European Patent Office
Office européen des brevets

(11) Publication number: 0 139 480 B1

(12) **EUROPEAN PATENT SPECIFICATION**

(45) Date of publication of patent specification: **11.12.91** (51) Int. Cl.[5]: **A61K 35/04**, A61K 7/48

(21) Application number: **84306415.5**

(22) Date of filing: **19.09.84**

# (54) Topical preparations containing tars and fatty acids.

(30) Priority: **29.09.83 GB 8326130**

(43) Date of publication of application:
**02.05.85 Bulletin 85/18**

(45) Publication of the grant of the patent:
**11.12.91 Bulletin 91/50**

(84) Designated Contracting States:
**AT BE CH DE FR GB IT LI LU NL SE**

(56) References cited:
**EP-A- 0 004 770**
**US-A- 4 178 373**

(73) Proprietor: **EFAMOL HOLDINGS PLC**
**Efamol House Woodbridge Meadows**
**Guildford Surrey GU1 1BA(GB)**

(72) Inventor: **Horrobin, David Frederick**
**P.O. Box 10**
**Nuns'Island Montreal, H3E 1J8(CA)**

(74) Representative: **Miller, Joseph et al**
**J. MILLER & CO. Lincoln House 296-302 High Holborn**
**London WC1V 7JH(GB)**

EP 0 139 480 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Tar preparations have been used in dermatology for well over a hundred years and still have a major place in the treatment of such conditions as eczema, psoriasis and other inflammatory and pruritic conditions of the skin. The mechanism of action of the tars is unknown.

The inventor has recently analysed blood samples of seven patients with severe psoriasis before and after a course of treatment with tars. The method of determinations was: Plasma samples (1ml) were extracted with chloroform:methanol (2:1). The extract was filtered through sodium sulphate, evaporated to dryness, and taken up in 0.5 ml chloroform:methanol. The lipid fractions were separated by thin layer chromatography on silica gel plates. The phospholipid fraction, taken to reflect essential fatty acid changes most sensitively, was methylated using boron trifluoride-methanol. The resulting methyl esters of the fatty acids were separated and measured using a Hewlett Packard 5880 gas chromatograph with a 180cm (six foot) column packed with 10% silar on Chromosorb WAW 106/230.® The carrier gas was helium (30 mL/min.). Oven temperature was programmed to rise from 165°C to 190°C at 2°C/min. Detector temperature was 220°C and injector temperature 200°C. Retention times and peak areas were automatically computed by a Hewlett Packard Level 4 integrator. Peaks were identified by comparison with standard fatty acid methyl esters.

The outstanding change in the blood was a rise in the level of dihomo-γ-linolenic acid (DGLA) in the plasma from below normal to above normal levels. The normal level of DGLA in human plasma phospholipids, expressed as a percentage of the total fatty acids present, is 3.06 ± 0.60. In the patients with psoriasis, before treatment with tar, the level was 2.66 ± 1.17. After treatment, the DGLA level was 4.03 ± 1.01, a statistically significant increase.

The inventor believes that the increase is significant in relation to the therapeutic action of the tars. DGLA gives rise in the body to prostaglandin $E_1$, a substance which has a wide range of desirable effects including the control of several forms of inflammaticn. This control, and the conversion of DGLA to prostaglandins, are discussed for example in EP-A-0 004 770 (EFAMOL) to which reference may be made. Specifically, a rise in plasma DGLA has desirable effects in the treatment of skin disorders such as eczema and psoriasis, and once such a rise has been found to be an effect of tar treatment it suggests that the value of the treatment may be enhanced by giving DGLA or other materials which give local or systemic rises in DGLA levels.

Thus according to the invention, use of dermatological tars and tar-based preparations (herein referred to generally as tars) has an enhanced therapeutic value if it is combined with use of dihomo-γ-linolenic acid (DGLA) or its precursor γ-linolenic acid (GLA), as such or as active derivatives as detailed later herein. The tars may be used in the ordinary way and the fatty acids either topically with the tars or as oral or other preparations acting through the bloodstream.

Primarily the invention lies in a product for topical application which comprises:

1. A dermatological tar or tar-based preparation;
2. γ-linolenic acid or dihomo-γ-linolenic acid, whether from natural or synthetic sources, as such or in the form of an active derivative thereof.

The product may be used in a method for treatment of inflammatory and pruritic conditions of the skin wherein such a tar or tar preparation is applied in conjunction with systemic or topical use of such acid(s) as derivatives. The invention further provides a pack comprising a topical dermatological tar or tar-based preparation and an oral form of GLA or DGLA or derivative thereof, presented separately but for use together.

Very many tar or tar-based preparations are available. Most are prepared from coal tar, some from pine tar (also known as Stockholm tar) and some from beech, birch or other wood tars. The chief components of such tars are pitch, benzene, naphthalene, phenols, pyridine and quinoline. The invention may therefore make use for example of Coal Tar BPC, Prepared Coal Tar BP, Tar BP, Pine Tar, Beech Tar or Birch Tar, or any preparation derived from coal or wood tar.

The essential fatty acids may be used as such or in the form of derivatives, particularly natural glyceride esters but also simple esters such as $C_1$ - $C_4$ alkyl esters, salts, amides and phospholipids. Such derivatives, having the effect of the acids themselves and pharmacologically acceptable, are referred to as active derivatives herein. Indirect identification of useful derivatives is by their having the valuable effect in the body of the acid itself, but conversion can be shown directly by gas chromatographic analysis of concentrations in blood, body fat, or other tissue by standard techniques for example those of Pelick et al p. 23, "Analysis of Lipids and Lipoproteins" Ed. Perkins, American Oil chemists Society, Champaign, Illinois, U.S.A. The most convenient derivative is the oil of the seed of the Evening Primrose (Oenothera spp) the analysis of which is given for example in EP-A-0 004 770 referred to above, containing γ-linolenic acid in

glyceride form.

The two components may be combined together in an appropriate topical form such as an ointment, paste, cream, lotion, soap, shampoo or emulsion according to methods in themselves standard in the art or as previously noted they may be used separately.

Examples of products are as follows, all by weight:

1. Coal tar cream: coal tar 2%, 'Cetamacrogol 1000'® 5%, isopropyl myristate 22%, wool fat 15%, emulsifying wax 5%, and evening primrose oil, safflower oil or borage oil 2% (or alternatively synthetic GLA or DGLA 0.2%), with water to 100%.

2. Coal tar paste: coal tar solution 5%, evening primrose oil 3%, soft paraffin to 100%.

3. Coal tar ointment: coal tar solution 3%, evening primrose oil 4%, wool alcohols ointment to 100%.

The range of concentration of the tars in such a product is suitably 0.01 to 30%, preferably 1 to 10%, by weight. The range of concentration of evening primrose oil is suitably 0.01 to 30%, preferably 0.2 to 5%, by weight with molar corresponding amounts of the fatty acids themselves or of other derivatives taking the GLA content of evening primrose oil as 9%, by weight.

The combination of tar and essential fatty acids can be used with other agents conventionally used for skin treatment, such as zinc compounds, salicylates, sulphur, hydrocortisone or other steroids, iodohydroxyquinoline, lauryl sulphate, resorcinol or benzoyl peroxide. All the products are applied daily in the same way as conventional coal tar preparations.

Further, essential fatty acids as a class have long been known to be important in skin function, and other essential fatty acids may with benefit be used in the products of the invention in addition to DGLA and GLA. Such acids include arachidonic acid (20:4n-6), adrenic acid (22:4n-6), and 22:5n-6, which will act by sparing DGLA from conversion in the bodily pathway from DGLA to arachidonic and the further acids and possibly by other mechanisms as yet unknown, and the essential fatty acids of the n-3 series, namely α-linolenic acid (18:3n-3), 18:4n-3, 20:4n-3, 20:5n-3, 22:5n-3 and 22:6n-3. Amounts may be as for DGLA and GLA.

There are sources of α-linolenic acid in a variety of vegetable oils such as soy and linseed oils, whereas the longer chain n-3 acids are found in some abundance in fish oils. Natural sources of arachidonic acid include for example egg yolks, and the higher n-6 acids can be obtained from adrenal glands and kidneys from slaughter houses.

The structures and relations of the acids are as follows:

| n-6 | n-3 | Structure |
|---|---|---|
| 18:2 | | $\Delta^{9,12}$ octadecadienoic acid |
| ↓ | | |
| 18:3 | 18:3 | $\Delta^{6,9,12}$ and $\Delta^{9,12,15}$ octadecatrienoic acids |
| ↓ | ↓ | |
| | 18:4 | $\Delta^{6,9,12,15}$ octadecatetraenoic acid |
| ↓ | ↓ | |
| 20:3 | | $\Delta^{8,11,14}$ eicosatrienoic acid |
| ↓ | ↓ | |
| 20:4 | 20:4 | $\Delta^{5,8,11,14}$ and $\Delta^{8,11,14,17}$ eicosatetraenoic acids |
| ↓ | ↓ | |
| | 20:5 | $\Delta^{5,8,11,14,17}$ eicosapentaenoic acid |
| ↓ | ↓ | |
| 22:4 | | $\Delta^{7,10,13,16}$ docosatetraenoic acid |
| ↓ | ↓ | |
| 22;5 | 22:5 | $\Delta^{4,7,10,13,16}$ and $\Delta^{7,10,13,16,19}$ docosapentaenoic acid |
| | ↓ | |
| | 22:6 | $\Delta^{4,7,10,13,16,19}$ docosahexaenoic acid |

The acids are in the natural all-cis configurations. In the n-6 series commonly used names for the 18:2, 18:3, 20:3, 20:4 and 22:4 acids are linoleic acid, $\gamma$-linolenic acid (GLA), dihomo-$\gamma$-linolenic acid (DGLA), arachidonic acid (AA) and adrenic acid. In the n-3 series only $\alpha$-linolenic acid (18:3) is commonly referred to by a non-systematic name.

The elongation stages in the natural metabolic pathway are much more rapid than the desaturations. The sequences, believed to be mediated by common enzymes in the two pathways, are:

| n-6 | | n-3 |
|---|---|---|
| 18:2 | | 18:3 |
| | $\Delta^{6}$ desaturase | |
| 18:3 | | 18:4 |
| | elongation | |
| 20:3 | | 20:4 |
| | $\Delta^{5}$ desaturase | |
| 20:4 | | 20:5 |
| | elongation | |
| 22:4 | | 22:5 |
| | $\Delta^{4}$ desaturase | |
| 22:5 | | 22:6 |

Studies of the interactions between the metabolism of the n-6 acids and that of the n-3 acids have shown that elongation reactions (e.g. GLA to DGLA) are highly efficient and there is very little competition either way. In contrast, the two series of fatty acids compete with one another effectively for the desaturation processes. This means that the n-3 fatty acids will interfere with both Δ-6 and Δ-5 desaturation in the n-6 series. This competition seems to occur even when the n-3 fatty acid is not actually a substrate for the enzyme concerned. For example, 20:5n-3 competitively inhibits Δ-6 desaturation. A consequence of this is that the presence of n-3 fatty acids in a combination will lead to some inhibition of the conversion of DGLA to arachidonic acid by the Δ-5-desaturase.

## Claims

### Claims for the following Contracting States: BE, FR, DE, IT, LU, NL, SE, CH, GB

1. A product for topical application against inflammatory and pruritic conditions of the skin which comprises a dermatological tar or tar-based preparation and gamma-linolenic acid (GLA) or dihomo-gamma-linolenic acid (DGLA) as such or in the form of a pharmacologically acceptable derivative therof having the effect of the acids themselves.

2. A product according to claim 1, comprising further arachidonic acid (20:4 n-6), adrenic acid (22:4 n-6) or docosapentaenoic acid (22:5 n-6) or an essential fatty acid of the n-3 series, namely alpha-linolenic acid (18:3 n-3) or the 18:4 n-3, 20:4 n-3, 20:5 n-3, 22:5 n-3 or 22:6 n-3 acid thereof.

3. A product according to claim 1 comprising further one or more of zinc compounds, salicylates, sulphur, hydrocortisone or other steroids, iodohydroxyquinoline, lauryl sulphate, resorcinol, or benzoyl peroxide, effective against said skin conditions.

4. A product according to claim 1 comprising 0.01 to 30%, preferably 1 to 10%, by weight, of said tar or tar-based preparation in said dermatological preparation.

5. A product according to claim 1, comprising 0.01 to 30%, preferably 0.2 to 5%, by weight, of evening primrose oil or molar corresponding amounts of GLA, DGLA or said derivative thereof (on the basis of a GLA content of evening primrose oil of 9%).

6. A pack comprising a topical dermatological tar or tar-based preparation and an oral form of GLA or DGLA or derivative thereof, presented separately but for use together.

7. A method of preparing a medicament for use in conjunction with a dermatological tar or tar-based preparation for treatment of inflammatory and pruritic conditions of the skin by application of the preparation to the skin, characterised in that gamma-linolenic acid or dihomo-gamma-linolenic acid or a pharmacologically acceptable derivative thereof having the effect of the acid itself is prepared as such medicament alone or with a pharmaceutical diluent or carrier for systemic or topical use in conjunction with the tar or tar-based preparation.

### Claims for the Contracting State: AT

1. A method of preparing a medicament for use in conjunction with a dermatological tar or tar-based preparation for treatment of inflammatory and pruritic conditions of the skin by application of the preparation to the skin, characterised in that gamma-linolenic (GLA) acid or dihomo-gamma-linolenic (DGLA) acid or a pharmacologically acceptable derivative thereof having the effect of the acid itself is prepared as such medicament alone or with a pharmaceutical diluent or carrier for systemic or topical use in conjunction with the tar or tar-based preparation.

2. A method according to claim 1, characterized in that the medicament is prepared to further comprise arachidonic acid (20:4 n-6), adrenic acid (22:4 n-6) or docosapentaenoic acid (22:5 n-6) or an essential fatty acid of the n-3 series, namely alpha-linolenic acid (18:3 n-3) or the 18:4 n-3, 20:4 n-3, 20:5 n-3, 22:5 n-3 or 22:6 n-3 acid thereof.

3. A method according to claim 1, characterized in that the medicament is prepared to further comprise one or more of zinc compounds, salicylates, sulphur, hydrocortisone or other steroids, iodohydroxyquinoline, lauryl sulphate, resorcinol, or benzoyl peroxide, effective against said skin conditions.

4. A method according to claim 1, characterized in that the medicament is prepared to comprise 0,01 to 30%, preferably 1 to 10%, by weight of said tar or tar-based preparation.

5. A method according to claim 1, characterized in that the medicament is prepared to comprise 0,01 to 30%, preferably 0,2 to 5%, by weight, of evening primrose oil or molar corresponding amounts of GLA, DGLA or said derivative thereof (on the basis of a GLA content of evening primrose oil of 9%).

**Revendications**
**Revendications pour les Etats contractants suivants: BE, FR, DE, IT, LU, NL, SE, CH, GB**

1. Produit pour application topique contre les pathologies dermatologiques inflammatoires et prurigineuses, comprenant une préparation dermatologique de goudron ou à base de goudron et de l'acide $\gamma$-linolénique (GLA) ou de l'acide dihomo-$\gamma$-linolénique (DGLA), en tant que tel ou sous la forme d'un dérivé pharmacologiquement acceptable, ayant l'effet des acides eux-mêmes.

2. Produit selon la revendication 1, comprenant en outre de l'acide arachidonique (20:4n-6), de l'acide adrénique (22:4n-6) ou de l'acide docosapentaènoïque (22:5n-6) ou un acide gras essentiel de la série n-3, à savoir l'acide $\alpha$-linolénique (18:3n-3) ou l'acide 18:4n-3, 20:4n-3, 20:5n-3, 22:5n-3 ou 22:6n-3 de cette série.

3. Produit selon la revendication 1, comprenant en outre une ou plusieurs des substances composés de zinc, salicylates, soufre, hydrocortisone ou autres stéroïdes, iodohydroxyquinoléine, laurylsulfate, résorcinol ou peroxyde de benzoyle, efficaces contre lesdites pathologies dermatologiques.

4. Produit selon la revendication 1, comprenant 0,01 à 30 %, de préférence 1 à 10 % en poids, de ladite préparation de goudron ou à base de goudron dans ladite préparation dermatologique.

5. Produit selon la revendication 1, comprenant 0,01 à 30 %, de préférence 0,2 à 5 % en poids d'huile d'onagre ou des quantités molaires correspondantes de GLA, DGLA ou dudit dérivé de ceux-ci (sur la base d'une teneur en GLA dans l'huile d'onagre de 9 %).

6. Unite comprenant une préparation dermatologique topique de goudron ou à base de goudron et une forme orale de GLA ou de DGLA ou d'un dérivé de ceux-ci, présentées séparément, mais destinées à être utilisées conjointement.

7. Procédé de préparation d'un médicament à utiliser en association avec une préparation dermatologique de goudron ou à base de goudron pour le traitement des pathologies dermatologiques inflammatoires et prurigineuses, par application de la préparation sur la peau, caractérisé en ce que l'acide $\gamma$-linolénique ou l'acide dihomo-$\gamma$-linolénique ou un dérivé pharmacologiquement acceptable de ceux-ci ayant l'effet de l'acide même est préparé en tant que médicament, seul ou avec un diluant pharmaceutique ou un véhicule pour usage systémique ou topique, en association avec la préparation de goudron ou à base de goudron.

**Revendications pour l'Etat contractant suivant: AT**

1. Procédé de préparation d'un médicament à utiliser en association avec une préparation dermatologique de goudron ou à base de goudron pour le traitement des pathologies dermatologiques inflammatoires et prurigineuses, par application de la préparation sur la peau, caractérisé en ce que l'acide $\gamma$-linolénique (GLA) ou l'acide dihomo-$\gamma$-linolénique (DGLA) ou un dérivé pharmacologiquement acceptable de ceux-ci ayant l'effet de l'acide même est préparé en tant que médicament, seul ou avec un diluant pharmaceutique ou un véhicule pour usage systémique ou topique en association avec la préparation de goudron ou à base de goudron.

2. Procédé selon la revendication 1, caractérisé en ce que le médicament est préparé de façon à comprendre en outre de l'acide arachidonique (20:4n-6), de l'acide adrénique (22:4n-6) ou de l'acide docosapentaènoïque (22:5n-6) ou un acide gras essentiel de la série n-3, à savoir l'acide $\alpha$-linolénique

(18:3n-3) ou l'acide 18:4n-3, 20:4n-3, 20:5n-3, 22:5n-3 ou 22:6n-3 de cette série.

3. Procédé selon la revendication 1, caractérisé en ce que le médicament est préparé de façon à comprendre en outre une ou plusieurs des substances composés de zinc, salicylates, soufre, hydrocortisone ou autres stéroïdes, iodohydroxyquinoléine, laurylsulfate, résorcinol ou peroxyde de benzoyle, efficaces contre lesdites pathologies dermatologiques.

4. Procédé selon la revendication 1, caractérisé en ce que le médicament est préparé de façon à comprendre 0,01 à 30 %, de préférence 1 à 10 % en poids, de ladite préparation de goudron ou à base de goudron.

5. Procédé selon la revendication 1, caractérisé en ce que le médicament est préparé de façon à comprendre 0,01 à 30 %, de préférence 0,2 à 5 % en poids d'huile d'onagre ou des quantités molaires correspondantes de GLA, DGLA ou dudit dérivé de ceux-ci (sur la base d'une teneur en GLA dans l'huile d'onage de 9 %).

**Patentansprüche**
**Patentansprüche für folgende Vertragsstaaten: BE, FR, DE, IT, LU, NL, SE, CH, GB**

1. Produkt für die örtliche Anwendung gegen entzündliche und pruriginöse Zustände der Haut, das einen dermatologischen Teer oder eine auf Teer basierende Zubereitung enthält und Gamma-Linolensäure (GLA) oder Dihomo-Gamma-Linolensäure (DGLA) als solche oder in Form eines pharmakologisch akzeptablen Derivates davon, das die Wirkung der eigentlichen Säuren aufweist.

2. Produkt nach Anspruch 1, das weiterhin Arachidon-Säure (20:4 n-6), Adren-Säure (englisch: adrenic acid) (22:4 n-6) oder Docosapentän-Säure (22:5 n-6) oder eine essentielle Fettsäure der n-3 Serie umfaßt, nämlich Alpha-Linolen-Säure (18:3 n-3) oder der 18:4 n-3, 20:4 n-3, 20:5 n-3, 22:5 n-3 oder 22:6 n-3 Säure davon.

3. Produkt nach Anspruch 1, das weiterhin ein oder mehrere Zinkbestandteile, Salicylate, Schwefel, Hydrocortison oder andere Steroide, Iodohydroxyquinolin (englisch: iodohydroxyquinoline), Lauryl-Sulfat, Resorcin, oder Benzoylperoxid umfaßt, die gegen besagte Hautzustände wirksam sind.

4. Produkt nach Anspruch 1, das 0,01 bis 30 Gew.-%, vorteilhaft 1 bis 10 Gew.-%, von besagtem Teer oder auf Teer basierenden Zubereitung in besagter dermatologischer Zubereitung enthält.

5. Produkt nach Anspruch 1, das 0,01 bis 30 Gew.-%, vorteilhaft 0,2 bis 5 Gew.-%, Nachtkerzenöl enthält oder entsprechende molare Mengen von GLA, DGLA oder besagtem Derivat davon (auf der Basis von einem GLA-Gehalt von Nachtkerzenöl von 9 %).

6. Packung, die einen örtlichen dermatologischen Teer oder eine auf Teer basierende Zubereitung und eine orale Form von GLA oder DGLA oder Derivaten davon enthält, die getrennt angeboten, aber zusammen verwendet werden.

7. Verfahren zur Zubereitung eines Medikamentes zur Verwendung in Verbindung mit einem dermatologischen Teer oder einer auf Teer basierenden Zubereitung zur Behandlung von entzündlichen und pruriginösen Hautzuständen durch Auftragen der Zubereitung auf die Haut, dadurch gekennzeichnet, daß Gamma-Linolen-Säure oder Dihomo-Gamma-Linolen-Säure oder ein pharmakologisch akzeptables Derivat davon mit der Wirkung der Säure selbst als ein derartiges Medikament allein zubereitet wird oder mit einem pharmazeutischen Verdünnungsmittel oder Trägersubstanz für die systemische oder örtliche Verwendung in Verbindung mit dem Teer oder einer auf Teer basierenden Zubereitung.

**Patentansprüche für folgenden Vertragsstaat: AT**

1. Verfahren zur Zubereitung eines Medikamentes, das in Verbindung mit einem dermatologischen Teer oder einer auf Teer basierenden Zubereitung für die Behandlung von entzündlichen und pruriginösen Zuständen der Haut durch Auftragen der Zubereitung auf die Haut verwendet wird, dadurch gekennzeichnet, daß Gamma-Linolensäure (GLA) oder Dihomo-Gamma-Linolen-Säure (DGLA) oder ein pharmakologisch akzeptables Derivat davon, das die Wirkung der Säure selbst aufweist, als ein derartiges Medikament allein oder mit einem pharmazeutischen Verdünnungsmittel oder Trägersubstanz für die systemische oder örtliche Verwendung in Verbindung mit dem Teer oder der auf Teer basierenden Zubereitung zubereitet wird.

2. Verfahren nach Anspruch 1, dadurch gekennzeichnet, daß das zubereitete Medikament weiterhin Arachidon-Säure (20:4 n-6), Adren-Säure (englisch: adrenic acid) (22:4 n-6) oder Docosapentän-Säure (22:5 n-6) oder eine essentielle Fettsäure der n-3 Serie umfaßt, nämlich Alpha-Linolen-Säure (18:3 n-3) oder der 18:4 n-3, 20:4 n-3, 20:5 n-3, 22:5 n-3 oder 22:6 n-3 Säure davon.

3. Verfahren nach Anspruch 1, dadurch gekennzeichnet, daß das Medikament zubereitet wird, so daß es weiterhin eine oder mehrere Zinkbestandteile, Salicylate, Schwefel, Hydrocortison oder andere Steroide, Iodohydroxyquinolin (englisch: iodohydroxyquinoline), Laurylsulfat, Resorcin, oder Benzoylperoxid umfaßt, die gegen besagte Hautzustände wirksam sind.

4. Verfahren nach Anspruch 1, dadurch gekennzeichnet, daß das Medikament zubereitet wird, so daß es 0,01 bis 30 Gew.-%, vorteilhaft 1 bis 10 Gew.-%, besagten Teeres oder auf Teer basierender Zubereitung enthält.

5. Verfahren nach Anspruch 1, dadurch gekennzeichnet, daß das Medikament zubereitet wird, so daß es 0,01 bis 30 Gew.-%, vorteilhaft 0,2 bis 5 Gew.-%, vom Öl der Nachtkerze enthält, oder entsprechende molare Mengen von GLA, DGLA oder besagten Derivaten davon (auf der Basis von einem GLA-Gehalt von Nachtkerzenöl von 9 %).